# EUROPEAN PATENT SPECIFICATION

(11) **EP 2 740 739 B1**
(45) Date of publication and mention of the grant of the patent: **28.12.2016**
(21) Application number: 11870457.6
(22) Date of filing: 24.10.2011
(51) Int. Cl.: C07K 14/375, C07K 1/22, C12N 15/31, C12N 15/63, C12N 1/00, A61K 38/16, A61P 35/00, G01N 33/50

(54) **AGROCYBE AEGERITA LECTIN AAL-2, AND ENCODING GENE THEREOF, PREPARATION METHOD THEREFOR AND APPLICATION THEREOF**
AGROCYBE AEGERITA LEKTIN AAL-2, UND DAVON KODIERENDE GEN, VORBEREITUNG VERFAHREN DAFÜR UND ANWENDUNG DAVON
LECTINE D'AGROCYBE AEGERITA AAL-2, ET GÈNE CODANT POUR CELLE-CI, PROCÉDÉ DE PRÉPARATION POUR CELLE-CI ET APPLICATION DE CELLE-CI

(30) Priority: 01.08.2011 CN 201110218000
(43) Date of publication of application: 11.06.2014
(73) Proprietor: Wuhan Alpha-Omega Biology Technology Co., Ltd., Wuhan, Hubei 430075 (CN)
(72) Inventor: SUN, Hui, Wuhan Hubei 430075 (CN); JIANG, Shuai, Wuhan Hubei 430075 (CN); GU, Bianli, Wuhan Hubei 430075 (CN); CHEN, Yijie, Wuhan Hubei 430075 (CN); YU, Guojun, Wuhan Hubei 430075 (CN); YIN, Yalin, Wuhan Hubei 430075 (CN); ZHANG, Jinlin, Wuhan Hubei430075 (CN)
(74) Representative: Stiebe, Lars Magnus
(86) International application number: PCT/CN2011/081164
(87) International publication number: WO 2013/016896

(56) References cited:
- YANG N ET AL: "Structural Basis for the Tumor Cell Apoptosis-Inducing Activity of an Antitumor Lectin from the Edible Mushroom Agrocybe aegerita", JOURNAL OF MOLECULAR BIOLOGY, vol. 387, no. 3, 3 April 2009 (2009-04-03) , pages 694-705, XP025982267, ACADEMIC PRESS, UNITED KINGDOM ISSN: 0022-2836, DOI: 10.1016/J.JMB.2009.02.002 [retrieved on 2009-02-09]
- ZHAO C ET AL: "An antitumour lectin from the edible mushroom Agrocybe aegerita", BIOCHEMICAL JOURNAL, vol. 374, 1 September 2003 (2003-09-01), pages 321-327, XP003032529, BIOCHEMICAL SOCIETY, LONDON, GB ISSN: 0006-2936
- YANG N ET AL: "Crystallization and preliminary crystallographic studies of the recombinant antitumour lectin from the edible mushroom Agrocybe aegerita", BIOCHIMICA ET BIOPHYSICA ACTA (BBA) - PROTEINS & PROTEOMICS, vol. 1751, no. 2, 10 August 2005 (2005-08-10), pages 209-212, XP027627734, ELSEVIER, NETHERLANDS ISSN: 1570-9639 [retrieved on 2005-08-10]
- DATABASE WPI Week 201112 Thomson Scientific, London, GB; AN 2011-B24510 XP002734313, -& CN 101 928 335 A (UNIV WUHAN) 29 December 2010 (2010-12-29)
- SHUAI JIANG ET AL: "A novel lectin from Agrocybe aegerita shows high binding selectivity for terminal N -acetylglucosamine", BIOCHEMICAL JOURNAL, vol. 162, no. 2, 15 April 2012 (2012-04-15), pages 1590-378, XP055161414, ISSN: 0264-6021, DOI: 10.1021/pr8007495
- SUN, HUI ET AL.: 'Purification and Characterization of a Novel Lectin AAVP from Fruiting Bodies of the Edible Fungus, Agrocybe aegerita' CHINESE JOURNAL OF BIOCHEMISTRY AND MOLECULAR BIOLOGY vol. 19, no. 1, February 2003, pages 96 - 102, XP003032527
- SUN, HUI ET AL.: 'Agrocybe aegerita Lectin AAVP with Antivirus and Mycelium Differentiation Promotion Activities' CHINESE JOURNAL OF BIOCHEMISTRY AND MOLECULAR BIOLOGY vol. 19, no. 2, April 2003, pages 210 - 214, XP003032528
- ZHAO, C. ET AL.: 'An antitumour lectin from the edible mushroom Agrocybe aegerita.' BIOCHEMICAL JOURNAL vol. 374, 01 September 2003, pages 321 - 327, XP003032529

### SEQUENCE LISTING

<110> Wuhan Alpha-Omega bioscience and biotechnology Co., Ltd.
<120> Agrocybe aegerita lectin AAL-2 and its encoding gene, the preparation methods and use thereof
<130> 140101-001KN
<140> PCT/CN2011/081164
   <141> 2011-10-24
<150> CN201110218000.5
   <151> 2011-08-01
<160> 8
<170> PatentIn version 3.5
<210> 1
   <211> 1224
   <212> DNA
   <213> Agrocybe aegerita
<400> 1
   atgaccagca acgtcatcac ccaagatttg cccatacccg tcgccagtcg cggctttgct      60
   gacattgttg gcttcggtct cgatggagtc gttatcgggc gcaacgccgt caaccttcag     120
   cccttccttg cggtcaagaa ctttgcacag aacgcaggag ggtggctcac cacgaaacac     180
   gttcgtctca ttgccgacac gaccggcacc ggaaaaggcg acatcgttgg gttcggcaac     240
   gctggcgtct atgtctctgt caataacggc aaaaacacgt ttgctgaccc gccgaagatg     300
   gtcattgcca acttcggtta cgatgcaggc ggctggcgcg tcgaaaagca ccttcgctac     360
   ttggccgaca tcaggaagat cggacgggct gatatcatag gttttgggga aaaaggagtc     420
   ctcgtctccc gcaacaacgg cggtttgaac ttcggccccg ccaccctcgt tttgaaagac     480
   tttggctacg atgccggtgg atggcgactc gaccgtcacc ttcgttttct tgctgacgtt     540
   acagggaacg gacacctcga catcgttggt ttcggtgaca agcacgtctt catcagccgt     600
   aacaacggcg atgggacttt cgcccccgca aaatctgtca tcgacaactt ctgcattgac     660
   gctggtggtt ggaagatcgg cgaccaccct cgcttcgtag ctgacttgac aggtgacgga     720
   acggccgaca tcattgggtg tggtaaggcc ggatgctggg tcgctctgaa taatggcggc     780
   ggcgtttttg ggcaggtcaa gttggttatc aacgactttg gaaccgataa gggttggcag     840
   gccgccaagc atcctcgatt cattgctgac ctcacgggca atgggcgcgg tgacgttgtt     900
   ggattcggga acgccggggt ctatgttgcg ctcaataacg gcgatggtac tttccagtcg     960
   gccaagctcg tgctcaagga cttcggcgtc caacagggat ggacggtcag caagcaccga    1020
   aggttcgtag tcgacttgac gggggacgga tgtgcagaca tcatcggctt tggagagaag    1080
   gagacactcg tctcgtacaa cgacggcaaa ggcaatttcg gcccagtcaa ggcccttacc    1140
   aacgactttt cgttcagcgg tggaaaatgg gccccggaga cgactgtctg ctggatggcc    1200
   aacttggaca gcagcaggca ttag                                            1224
<210> 2
   <211> 407
   <212> PRT
   <213> Agrocybe aegerita
<400> 2
   Met Thr Ser Asn Val Ile Thr Gln Asp Leu Pro Ile Pro Val Ala Ser
   1 5 10 15
   Arg Gly Phe Ala Asp Ile Val Gly Phe Gly Leu Asp Gly Val Val Ile
   20 25 30
   Gly Arg Asn Ala Val Asn Leu Gln Pro Phe Leu Ala Val Lys Asn Phe
   35 40 45
   Ala Gln Asn Ala Gly Gly Trp Leu Thr Thr Lys His Val Arg Leu Ile
   50 55 60
   Ala Asp Thr Thr Gly Thr Gly Lys Gly Asp Ile Val Gly Phe Gly Asn
   65 70 75 80
   Ala Gly Val Tyr Val Ser Val Asn Asn Gly Lys Asn Thr Phe Ala Asp
   85 90 95
   Pro Pro Lys Met Val Ile Ala Asn Phe Gly Tyr Asp Ala Gly Gly Trp
   100 105 110
   Arg Val Glu Lys His Leu Arg Tyr Leu Ala Asp Ile Arg Lys Ile Gly
   115 120 125
   Arg Ala Asp Ile Ile Gly Phe Gly Glu Lys Gly Val Leu Val Ser Arg
   130 135 140
   Asn Asn Gly Gly Leu Asn Phe Gly Pro Ala Thr Leu Val Leu Lys Asp
   145 150 155 160
   Phe Gly Tyr Asp Ala Gly Gly Trp Arg Leu Asp Arg His Leu Arg Phe
   165 170 175
   Leu Ala Asp Val Thr Gly Asn Gly His Leu Asp Ile Val Gly Phe Gly
   180 185 190
   Asp Lys His Val Phe Ile Ser Arg Asn Asn Gly Asp Gly Thr Phe Ala
   195 200 205
   Pro Ala Lys Ser Val Ile Asp Asn Phe Cys Ile Asp Ala Gly Gly Trp
   210 215 220
   Lys Ile Gly Asp His Pro Arg Phe Val Ala Asp Leu Thr Gly Asp Gly
   225 230 235 240
   Thr Ala Asp Ile Ile Gly Cys Gly Lys Ala Gly Cys Trp Val Ala Leu
   245 250 255
   Asn Asn Gly Gly Gly Val Phe Gly Gln Val Lys Leu Val Ile Asn Asp
   260 265 270
   Phe Gly Thr Asp Lys Gly Trp Gln Ala Ala Lys His Pro Arg Phe Ile
   275 280 285
   Ala Asp Leu Thr Gly Asn Gly Arg Gly Asp Val Val Gly Phe Gly Asn
   290 295 300
   Ala Gly Val Tyr Val Ala Leu Asn Asn Gly Asp Gly Thr Phe Gln Ser
   305 310 315 320
   Ala Lys Leu Val Leu Lys Asp Phe Gly Val Gln Gln Gly Trp Thr Val
   325 330 335
   Ser Lys His Arg Arg Phe Val Val Asp Leu Thr Gly Asp Gly Cys Ala
   340 345 350
   Asp Ile Ile Gly Phe Gly Glu Lys Glu Thr Leu Val Ser Tyr Asn Asp
   355 360 365
   Gly Lys Gly Asn Phe Gly Pro Val Lys Ala Leu Thr Asn Asp Phe Ser
   370 375 380
   Phe Ser Gly Gly Lys Trp Ala Pro Glu Thr Thr Val Cys Trp Met Ala
   385 390 395 400
   Asn Leu Asp Ser Ser Arg His
   405
<210> 3
   <211> 17
   <212> PRT
   <213> Agrocybe aegerita
<400> 3
   Gly Phe Ala Asn Ile Val Gly Phe Gly Leu Asp Gly Val Val Ile Gly
   1 5 10 15
   Arg
<210> 4
   <211> 14
   <212> PRT
   <213> Agrocybe aegerita
<400> 4
   Met Val Ile Ala Asn Phe Gly Tyr Asp Ala Gly Gly Trp Arg
   1               5                   10
<210> 5
   <211> 6
   <212> PRT
   <213> Agrocybe aegerita
<400> 5
   Tyr Leu Ala Asp Ile Arg
   1               5
<210> 6
   <211> 10
   <212> PRT
   <213> Agrocybe aegerita
<400> 6
   Asp Phe Gly Tyr Asp Ala Gly Gly Trp Arg
   1               5                   10
<210> 7
   <211> 6
   <212> PRT
   <213> Agrocybe aegerita
<400> 7
   His Val Phe Ile Ser Arg
   1               5
<210> 8
   <211> 10
   <212> PRT
   <213> Agrocybe aegerita
<400> 8
   Phe Ile Ala Asp Leu Thr Gly Asn Gly Arg
   1               5                   10

## Description

### FIELD OF THE INVENTION

This invention relates to a kind of lectin isolated from fungi and its encoding gene, particularly relates to the lectin AAL-2 isolated from the *Agrocybe aegerita* and its encoding gene. This invention also relates to a method for the isolation of the *Agrocybe aegerita* lectin AAL-2 and its uses in antitumor or detecting in vitro the N-Acetylglucosamine relevant diseases and the N-Acetylglucosamine relevant carbohydrate structure. This invention belongs to the application and separation field of *Agrocybe aegerita* lectin.

### BACKGROUND OF THE INVENTION

The medicinal fungus has more than two thousand years history in the East for its peculiar medicinal values (especially for its antitumor activity). Until the 1950s, as the maturing of the technology and the isolation and identification of the active components such as the polysaccharide etc, the medicinal value of the medicinal fungus is gradually taken seriously by the researchers in the United States and Europe. More and more active components of the medicinal fungus are screened out and confirmed in the animal test and clinical trials. The medicinal fungus becomes a treasure house for screening the antitumor drugs.

The *Agrocybe aegerita* is also named *Agrocybe cylindracea*, *Agrocybe aegerita, Liu Songrong* etc, which is subordinated to Eumycetes, Basidiomycetes, Agaricales, Bolbitiaceae, Agrocybe. The *Agrocybe aegerita* has 8 kinds of the body essential ammonia acid, wherein the content of the lysine is high up to 1.75%. The nutritional values of the *Agrocybe aegerita* exceed that of other edible mushrooms and it has peculiar medicinal values. Peoples usually use it for anticancer, lowing the blood pressure, treating stomach cold, nephritis, edema and so on, which has distinctive curative effects. But the pharmacological components are not clear.

The primary liver cancer is a kind of the most common malignant tumour in the world, the incidence of disease and the death ratio of which are the seventh and the third in the world. In china the incidence of liver cancer is 25.7/10 million after the lung cancer and stomach cancer, the death rate of which is high up to 23.7/10 million and which is the second in the china cancer mortality rate.Now the acknowledged diagnosis and therapy are the ultrasonic diagnosis, the percutaneous ethanol injection, the radiotherapy and chemotherapy and the operation etc. But the reoccurrence rate and transfer rate of the liver cancer are much higher. So it is urgently needed to search medicines and methods to inhibite the liver cancer more effectively or kill the liver cancer cells.

Lectin is a kind of nonimmune original protein widespread in nature, which can reversibly combine a variety of carbohydrates or glycoproteins and the combination is different from that of the enzyme with the substrate.Till now, humans have isolated hundreds of lectins and each lectin has its peculiar biochemical character, carbohydrate binding specificity and broad biological activity such as anticancer activity, antimycotic activity and antiviral activity, etc. An antitumour lectin AAL was isolated from the total protein of the *Agrocybe aegerita* using a procedure which involved ion exchange chromatography by Hui SUN, etc. AAL showed strong inhibition of the growth of human tumour cell lines including humane colonic adenocarcinoma, human cervical carcinoma, human stomach cancer, human promyelocytic leukaemia (HL-60) and so on, wherein the inhibition ratio of AAL in HL-60 cells was significantly higher than that of adriamycin. (Hui SUN,etc.An antitumour lectin from the edible mushroom Agrocybe aegerita. Biochem. J. (2003) 374, 321-327).

In Yang N. et al, "Structural Basis for the Tumor Cell Apoptoisis-Inducing Activity of an Antitumor Lectin from the Edible Mushroom Agrocybe aegerita" J. Mol. Bio. (2009) 387 (3), 694-705 a lectin AAL is disclosed. The authors disclose that dimerization of the AAL is a prerequisite for its tumor cell apoptosis-inducing activity. Also the hydrophobic pocket essential to its activity is identified. The investigations are based on the study of 11 alternative mutants.

### SUMMARY OF INVENTION

It is an object of the present invention to provide a new lectin isolated from the *Agrocybe aegenita.*

It is a further object of the present invention to provide a method for isolating or preparing the said lectin from the *Agrocybe aegerita.*

It is an additional object of the present invention to provide the cDNA edcoding the above isolated *Agrocybe aegerita* lectin.

The fourth object of the present invention is to provide the pharmacological uses of the *Agrocybe aegerita* lectin and its encoding gene.

The above objects of the invention are realized through the following technical schemes:

The present invention obtains a new lectin isolated from the *Agrocybe aegerita* total protein by affinity chromatography, named *Agrocybe aegerita* lectin AAL-2, of which the amino acid sequence is shown as the following (a) or (b):
(a) The amino acid sequence set forth in SEQ ID NO: 2; or
(b) The polypeptide variants obtained by the substitution, deletion or insertion of one or multiple amino.acid residues in the amino acid sequence set forth in SEQ ID NO: 2 still have the function of *Agrocybe aegerita* lectin AAL-2.

Preferably, the amino acid sequence of the *Agrocybe aegerita* lectin AAL-2 described in the present invention is as set forth in SEQ ID NO: 2.

The term "multiple" in this invention usually means 1-50, the better is 1-30, much better is 1-20, the best is 2-4, which is determined by the types of amino acids or the position of the amino acid residues in the three-dimensional structure of the lectin AAL-2. The term "substitution" means using different amino acid residues to substitute one or multiple amino acid residues. For example, in the art it usually does not change the protein function by using the property close or similar amino acid sequence for substitution. The term"deletion" means the changes of the amino acid sequence, lacking one or multiple amino acid residues, respectively. The term "insertion" means the change of the amino acid sequence, relatively to the natural molecules, the changes lead to adding one or multiple amino acid residues or adding one or multiple amino acids in the C-terminal and/or N-terminal(usually within 20, better within10, much better within 5 amino acids).

The polypeptide variants of the *Agrocybe aegerita* lectin AAL-2 described in the present invention including: homologous sequence variant, conservative variants, allelic variant, natural mutant, induced mutant, the proteins encoded by the DNA under high or low stringent hybridization conditions that can hybridize to the nucleotide sequence of the *Agrocybe aegerita* lectin AAL-2 and the polypeptides or proteins obtained by using the antiserum which anti the polypeptide of *Agrocybe aegerita* lectin AAL-2.

The "stringent hybridization conditions" means low ionic strength and high temperature conditions known in the art. Usually, under stringent conditions, a probe will hybridize to its target sequence to a detectably greater degree than to other sequences (e.g., at least 2-fold over background). Stringent hybridization conditions are sequence-dependent and will be different in different circumstances, longer sequences can specifically hybridize at higher temperature. By controlling the stringency of the hybridization or washing conditions, target sequences that are 100% complementary to the probe can be identified. The detailed guidance of nucleic acid hybridization can refer to the relevant literature (Tijssen, *Techniques in Biochemistry and Molecular Biology-Hybridization with Nucleic Probes,* "Overview of principles of hybridization and the strategy of nucleic acid assay".1993).More specifically, stringent conditions are usually selected to be about 5-10°C lower than the thermal melting point (Tm) for the specific sequence and its complement under defined ionic strength and pH. Tₘ is the temperature, in the equilibrium state, when 50% probes complementary to the targets hybridizes to the target sequence (under the specified ionic strength, pH and nucleic acid concentration) (because the target sequence is present in excess, so 50% of the probe under the Tₘ in the equilibrium state is occupied). Stringent conditions can be the following conditions: the salt concentration is less than about 1.0 M Na ion concentration at pH 7.0 to 8.3, typically about 0.01 to 1.0 M Na ion concentration (or other salts) and the temperature for short probes (including (but not limited to) 10 to 50 nucleotides) is at least about 30°C and for long probes (including (but not limited to) greater than 50 nucleotides) is at least about 60°C. Stringent conditions also can be achieved with the addition of destabilizing agents such as formamide. For a selective or specific hybridization, the positive signal is at least twice the background hybridization, or 10 times of the background hybridization according to the case. As a reference, the "stringent hybridization conditions" can be summarized as follows: 50% formamide, 5 × SSC and 1% SDS, incubated at 42 °C; or 5 x SSC, 1% SDS, cultured at 65 °C, washing in 0.2x SSC and at 65°C a wash in 0.1% SDS. The said wash can be carried out for 5, 15, 30, 60, 120 minutes or longer.

The polypeptide variants of the invention can be generated by genetic polymorphisms or manual operation, where the operation method is usually known in the art. For example, the variants or fragments of the amino acid sequence as set forth in SEQ ID NO: 2 can be prepared through the DNA mutation, of which the method for inducing mutation or changing polynucleotide is known for ordinary technical personnel in this art.

The invention also provides other polypeptides, such as a fusion protein composed of *Agrocybe aegerita* lectin AAL-2 or fragments thereof.

The invention also includes the analogs of the *Agrocybe aegerita* lectin AAL-2 protein or polypeptide. The difference between these analogues and natural *Agrocybe aegerita* lectin AAL-2 polypeptide can be differences in the amino acid sequence, or differences of modified forms that do not affect the amino acid sequence, or containing both of the above two at the same time.

These polypeptides include natural or induced genetic variants. Induced variants can be achieved through a variety of techniques, such as by radiation or exposure to mutagens producing random mutation, also by the site-directed mutagenesis technique or other known techniques in molecular biology. Analogues also include analogues of which the residues are different from natural L-amino acid (such as D-amino acid) and analogues with non natural survival or synthetic (such as β, γ-amino acids) amino acid. The modified (usually do not change the primary structure) forms include that: the chemical derived forms of the polypeptide in vivo or in vitro, such as acetylation or carboxylated. The modification also includes glycosylation, such as those polypeptides produced by glycosylation modification in the synthesis and the processing of the polypeptide or further processing steps. This modification can be achieved through exposing the polypeptide to the glycosylation enzymes (such as glycosylation enzymes or deglycosylation enzymes in mammalian). The modified forms also include the sequence that has the phosphorylated amino acid residues (such as phosphotyrosine, phosphoric acid threonine, serine phosphate). Also included are modified polypeptides improving the resistance to proteolysis performance or optimized the dissolution property.

The second object of this invention is to provide a method to isolate the *Agrocybe aegerita* lectin AAL-2 from the *Agrocybe aegerita,* including the following steps of:(1) extracting the total protein of *Agrocybe aegerita* and removing denatured proteins; (2) after removing the denatured proteins, the total protein of the *Agrocybe aegerita* passing by the affinity column and washing the affinity column with buffer until the ultraviolet detector detected no other proteins outflow; (3) eluting affinity column using buffer containing N-Acetylglucosamine and collecting the effluent proteins, namely AAL-2.

In order to achieve a much better isolation effect, preferentially select the N-Acetylglucosamine conjugated Sepharose 6B as the affinity column; the buffer in step (2) is the PBS buffer solution; in step (3) the preferred is eluting with PBS buffer containing 0.2mol/L N-Acetylglucosamine.

The present invention uses the affinity chromatography in the process of separation and purification of the *Agrocybe aegerita* lectin AAL-2 by using the N-Acetylglucosamine conjugated Sepharose-6B affinity column and isolates the lectin AAL-2 from the *Agrocybe aegerita* total protein by a step. In this invention, the method for isolating the *Agrocybe aegerita* lectin AAL-2 has the advantages of simple process, sample of high purity and low preparation cost.

The third object of the present invention is to provide the encoding nucleotide sequence of the said lectin AAL-2.

The cDNA sequence encoding the *Agrocybe aegerita* lectin AAL-2 is the following (a) or (b) nucleotide sequence:
(a) The nucleotide sequence set forth in SEQ ID NO : 1; or
(b)The nucleotide variants obtained by one or multiple bases substitution, deletion or/and insertion in the nucleotide sequence set forth in SEQ ID NO: 1, while the protein encoded by which still has the activity or function of the *Agrocybe aegerita* lectin AAL-2.

The nucleotide variation forms include but do not limited to: usually 1-90, the better 1-60, much better 1-20, the best 1-10 nucleotides of deletion, insertion and/or substitution, and the variants obtained by adding multiple nucleotides to the 5'and/or 3'terminal (usually within 60, the better for within 30, much better within10, the best within 5).

The "nucleotide variants" refer to the basically similar sequences. To the polynucleotide, the variants contain the deletion, insertion or/and substitution of one or multiple nucleotides in one or multiple sites of the natural polynucleotide. To the polynucleotide, conservative variants include the variants that owing to the degeneracy of the genetic code while without changing the amino acid sequences. And such naturally occurring variants can be identified through molecular biology techniques available. Variant polynucleotide also includes synthesis sourced polynucleotide, such as the polynucleotide variants obtained by the site-directed mutagenesis technique that still encode the amino acid set forth in the SEQ ID No.2, or by recombinant methods (such as DNA shuffling). The technicians in this art can use the following molecular biology technologies to screen or evaluate the function or activity of the proteins encoded by the polynucleotide variants: c arbohydrate binding activity, DNA binding activity, protein interactions, instantaneous in the activation of gene expression and so on.

The invention also extends to an expression vector comprising the cDNA sequence encoding the *Agrocybe aegerita* lectin AAL-2 as defined above and host cells containing the above expression vector.

The cDNA sequence encoding the *Agrocybe aegerita* lectin AAL-2 is inserted into a expression vector (expression vector as described can be a variety of commercially available expression vectors, including plasmid, clay etc) between appropriate restriction enzyme cutting sites and operationally connected with expression and regulation sequence, then the expression vector expressing the cDNA sequence is obtained; the constructed expression vector can be composed by the 5' non coding region, cDNA sequence as set forth in SEQ ID NO: land 3' non coding region, among which the 5' non coding region may include promoter, enhancer or/and translation enhancer sequence; the promoter sequence described above can be constitutive, inducible, tissue or organ specific elicitors.

The 3' non coding region as described may contain the terminator sequence, mRNA cutting sequence and so on. The cDNA sequence as set forth in SEQ ID NO:1 can be replaced by the nucleotide variants obtained by one or multiple bases substitution, deletion or/and insertion in the cDNA sequence described above.The encoded protein of the nucleotide variants still has the function of the *Agrocybe aegerita* lectin AAL-2.

The invention also includes the antisense sequence of the cDNA sequence encoding the *Agrocybe aegerita* lectin AAL-2 polypeptide. This antisense sequence can be used to inhibit the intracellular expression of *Agrocybe aegerita* lectin protein AAL-2.

The invention also includes a nucleotide molecule that can be used as a probe, which usually has 8-100 nucleotides of the coding sequence of *Agrocybe aegerita* lectin AAL-2 polypeptide, preferably has15-50 continuous nucleotides. The probe can be used to detect in vitro the presence of the nucleic acid molecules that encode *Agrocybe aegerita* lectin protein AAL-2 in test sample.

The invention also includes a method for detecting in vitro the *Agrocybe aegerita* lectin AAL-2 nucleotide sequence, including that: the probe described above hybridizing to the sample, then detecting in vitro whether the probe is combined. Preferably, the samples are PCR amplified productions and the PCR primer corresponds to the coding sequence of the *Agrocybe aegerita* lectin AAL-2 polypeptide, which can be located in the both sides or in the middle of the coding sequence. The primer length is generally 20-50 nucleotides.

The invention also provides the specificity sourced polyclonal antibody and monoclonal antibody to the *Agrocybe aegerita* lectin protein AAL-2 or polypeptides encoded by the AAL-2 fragments. The term "specificity" refers to that the antibody can bind in vitro to the *Agrocybe aegerita* lectin AAL-2 gene products or fragments. Better, refers to the antibody that can combine with *Agrocybe aegerita* lectin *AAL-2* gene products or fragments while does not recognize and bind to other non related antigen molecules. In the invention the antibodies include the molecules capable of combining and inhibiting the *Agrocybe aegerita* lectin AAL-2. The invention also includes those antibodies that can combine with the *Agrocybe aegerita* lectin *AAL-2* gene products whether modified or not.

The invention includes not only whole monoclonal antibody or polyclonal antibody, but also includes the antibody fragments with immunological activity, such as Fab' or (Fab)₂ fragments, heavy chain of antibody, light chain of antibody, by genetic process engineered single stranded Fv molecule or chimeric antibody, such as an antibody that has the mouse antibody binding specificity while still retains the antibody part of the human.

The antibodies of the present invention can be prepared by various known techniques by the ordinary technical personnel in the art. For example, the purified *Agrocybe aegerita* lectin *AAL-2* gene products or the polypeptide fragments with antigenicity are applied to the animal to induce the polyclonal antibody. Similarly, the cell which expressing the *Agrocybe aegerita* lectin AAL-2 or its fragments with antigenicity can be used to produce antibody by immuning animals. An antibody of the invention also can be monoclonal antibody. The monoclonal antibody described above can be prepared by hybridoma technique. The antibodies of the invention include antibodies that can block the function of the *Agrocybe aegerita* lectin AAL-2 and that do not affect the function of the *Agrocybe aegerita* lectin AAL-2. All the antibodies of the invention can be obtained by conventional immunization techniques using the fragments or functional area of the *Agrocybe aegerita* lectin *AAL-*2 gene products. These fragments or functional areas can be prepared using the recombinant methods or using the peptide synthesizer. And the antibody binding to the unmodified form of the *Agrocybe aegerita lectin AAL-2* gene product can be produced by using the gene products produced in prokaryotic cells (such as E.coli) to immune animals; the antibody binding to the modified forms after the translation (such as glycosylation or phosphorylation of protein or polypeptide) can be produced by using the gene products produced in eukaryotic cells (such as yeast or insect cells) to immune animals.

The vitro cell experiment indicates that the isolated AAL-2 in the invention has inhibition activity to the tumor cells which can induce significantly apoptosis of the hepatoma cells and the apoptosis inducing activity depends on the concentration of the *Agrocybe aegerita* lectin AAL-2. At the same time, in the invention the mouse hepatoma H22 tumor is used to construct the tumor bearing mice model, by intratumoral injection of AAL-2, the tumor growth of the tumor bearing mice is significantly inhibited and the survival time of the tumor bearing mice is prolonged obviously, which show that AAL-2 has a better therapeutic effect on tumor.

When the *Agrocybe aegerita* lectin AAL-2 is used in the treatment of tumor, the dose and mode can be determined according to the factors of tumor types, the condition of the patient and so on. The *Agrocybe aegerita* lectin AAL-2 or its mutants can be prepared into injection, through intravenous injection, single or multiple tumors injection, intramuscular injection or intravenous injection, or be prepared into oral preparations such as enteric coated capsules, colon capsule, microsphere, nanoparticle and so on.

In addition, the nucleotide encoding *Agrocybe aegerita* lectin AAL-2 or its mutants (for example: SEQ ID No. 1) can be used to construct a expression vector suitable for expressing in human body in order to prepare a gene therapy drug and these methods are mastered by the technical staff in the art.

The invention also identifies the carbohydrate binding spectrum of the *Agrocybe aegerita* lectin AAL-2 and the results show that AAL-2 is a kind of protein which has high binding specificity to the N-Acetylglucosamine. The detection results of the glycochip technology indicate that the *Agrocybe aegerita* lectin AAL-2 preferentially bind to the carbohydrate or glycoprotein with the N-Acetylglucosamine as the terminal, which show that the *Agrocybe aegerita* lectin AAL-2 can be used in N-Acetylglucosamine relevant disease in vitro detection and as an agent detecting in vitro the N-Acetylglucosamine relevant carbohydrate structure, which have the potential to developing into a diagnostic probe.

### BRIEF DESCRIPTION OF THE FIGURES

Figure 1 The affinity diagram and SDS-PAGE electrophoresis results of the *Agrocybe aegerita* lectin AAL-2; (A). The affinity diagram of the *Agrocybe aegerita* lectin AAL-2;(B). SDS-PAGE electrophoresis results of Agrocybe aegerita lectin AAL-2; The lane 1 is the total protein of *Agrocybe aegerita*, lane 2 is the protein passing through the affinity column from *Agrocybe aegerita* total protein, lane 3 is *Agrocybe aegerita* lectins AAL-2, lane M is a protein Marker.
Figure 2 The homology degree comparison results of amino acid sequence of the *Agrocybe aegerita* lectin AAL-2 and that of the peptides obtained by mass spectrometry.
Figure 3 The homology degree comparison results of the nucleotide sequence of *Agrocybe aegerita* lectin AAL-2 and that of *Agrocybe aegerita* lectin AAL.
Figure 4 The homology comparison results of the amino acid sequence of the *Arocybe aegerita* lectin AAL-2 and the *Agrocybe aegerita* lectin AAL.
Figure 5 The SDS-PAGE electrophoresis results of the *Agrocybe aegerita* lectin AAL-2 and *Agrocybe aegerita* lectin AAL.
Figure 6 The experimental results of tumor cell apoptosis induced by *Agrocybe aegerita* lectin AAL-2: (A).AAL-2 induced H22 cells apoptosis; (B). The AAL-2 induced Huh7 cells apoptosis.
Figure 7 The experimental results of the inhibition effect of *Agrocybe aegerita* lectin AAL-2 to the tumor tissues growth of the tumor bearing mice; (A).AAL-2 inhibits tumor growth; (B).AAL-2 prolongs the survival period of tumor bearing mice.

### Example 1 The preparation of Agrocybe aegerita lectin AAL-2 protein

### (1).The preparation of the Agrocybe aegeruta total protein

The edible mushroom *Agrocybe aegeruta* is collected from Sanming Institute of Fungi (Sanming, Fujian, P. R. China). Dry fruiting bodies are crushed into a powder, and according to a 1:10 (w/v) proportion this powder is extracted thrice with distilled water for 4h each time or a whole night. Solid ammonium sulphate is added to the supernatant to 80% saturation, keeping static overnight. The extract is combined and centrifuged at 10 000 g for 20 minutes. The precipitate is collected, dissolved in a small volume of distilled water and dialysed extensively. After freeze-drying, the total protein of the *Agrocybe aegeruta* is obtained.

### (2). Separation and purification by the affinity column

400 mg total protein of *Agrocybe aegerita* is dissolved in 90 ml PBS buffer solution and centrifuged to remove the denatured protein. The affinity chromatography is performed on N-Acetylglucosamine conjugated Sepharose 6B affinity column on which the supernatant is applied. After balanced with the PBS buffer, the supernatant of *Agrocybe aegerita* total protein passes by the affinity column till the sample is completed. Wash the affinity column with PBS buffer until the ultraviolet detector detectes no other proteins outflow. Elute the affinity column with the PBS buffer containing 0.2 mol/L of N-Acetylglucosamine and collect the effluent proteins, namely *Agrocybe aegerita* lectin AAL-2.

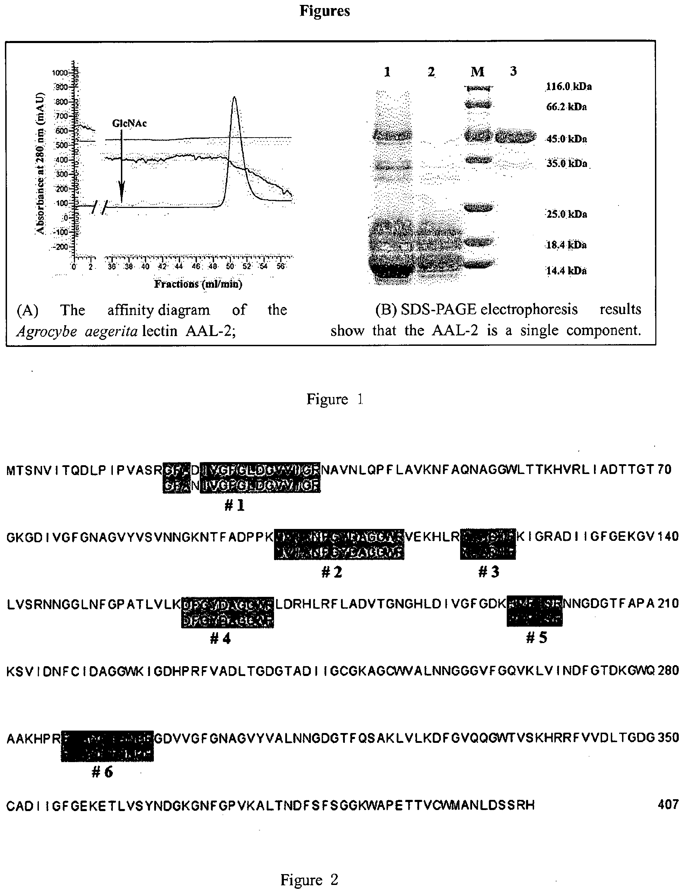

Remove the redundant N-Acetylglucosamine from the collected AAL-2 by dialysis and detect the coagulation activity of AAL-2 using the chicken erythrocyte. SDS-PAGE detection indicates that the *Agrocybe aegerita* lectin AAL-2 is a single protein component as shown in Figure 1.

### Example 2 Determination of the nucleotide coding region of Agrocybe aegerita lectin AAL-2 protein and the amino acid code of the AAL-2 protein

Cut the AAL-2 band obtained by SDS-PAGE in example 1 and identified by the mass spectrometry, 6 peptides are got. At the same time, in the present invention the transcriptome libraries of the *Agrocybe aegerita* mycelium and fruiting body are constructed (NCBI code is SRA026731) and homology comparison of the amino acid sequences of 6 peptides with the transcriptome library is performed by the local BLASTX. Comparisons show that the nucleotide coding region of AAL-2 protein locates on the complementary strand of gene *Unigene4198* in the transcriptome library. By confirming the open reading frame, a protein coding region is obtained. The amino acid sequence translated by the coding region of the protein can be matched with the 6 peptides of AAL-2 obtained by the mass spectrometry (Figure 2). The predicted protein molecular weight is 43175 kDa using the ExPASy software, of which the results are consistent with the SDS-PAGE results in example 1. So this coding region is believed to be the nucleotide sequence of *Agrocybe aegerita* lectin AAL-2, the nucleotide sequence is shown in SEQ ID No.1. It is confirmed by mass spectrometry identification that the encoded amino acid sequence is set forth in SEQ ID No.2.

**Table 1. amino acid sequence of 6 AAL-2 enzyme digested peptides by mass spectrometric analysis**

| the peptide number | amino acid sequence |
|---|---|
| **#1** | **GFANIVGFGLDGVVIGR** |
| **#2** | **MVIANFGYDAGGWR** |
| **#3** | **YLADIR** |
| **#4** | **DFGYDAGGWR** |
| **#5** | **HVFISR** |
| **#6** | **FIADLTGNGR** |

### Experimental example 1 Homology analysis of the Agrocybe aegerita lectin AAL-2 seperated in the present invention and the published Agrocybe aegerita lectin AAL

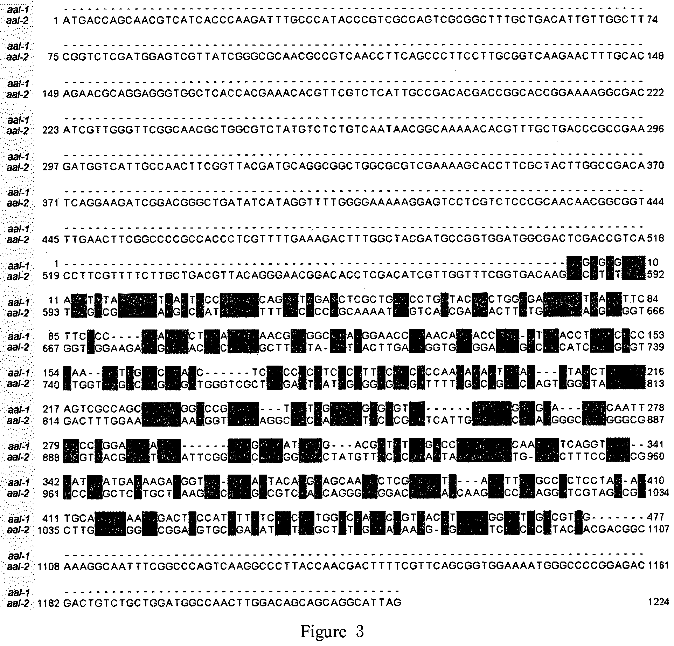

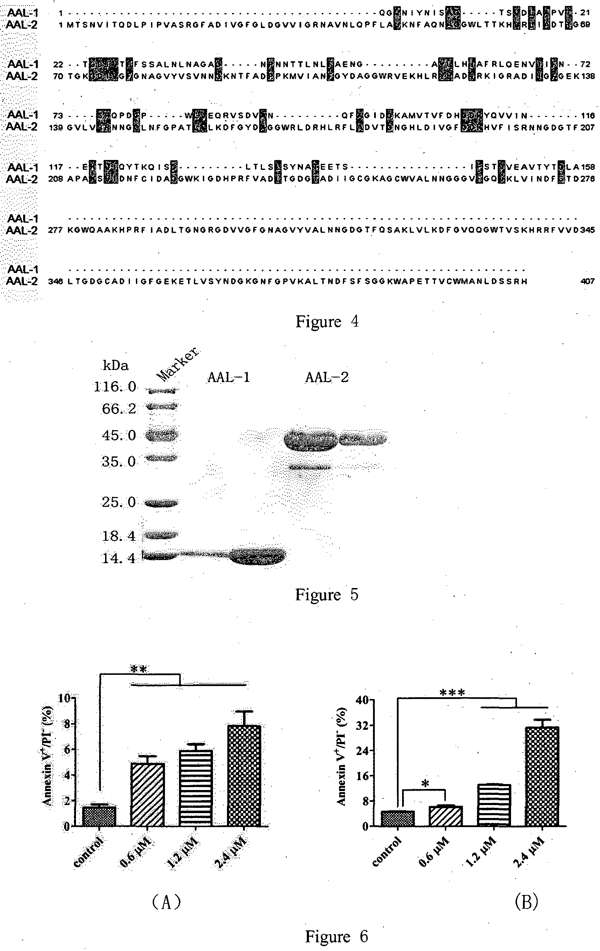

1.The nucleotide homology comparison and analysis results of the separated *Agrocybe aegerita* lectin AAL-2 in this invention and the published *Agrocybe aegerita* lectin AAL are shown in Figure 3. From the comparison results, we can see that the homology of the two nucleotides is 40.63% (193/475), Gap=61.22% (750/1225).
2. The amino acid homology analysis results of the separated *Agrocybe aegerita* lectin AAL-2 in this invention and the published *Agrocybe aegerita* lectin AAL are shown in Figure 4. From the comparison results, we can see that the amino acid homology is 22.78% (36/158) and the similarity of amino acid residues is 47.47% (75/158), Gap= 61.18% .(249/407).
3. The SDS-PAGE results of the *Agrocybe aegerita* lectin AAL-2 and the published *Agrocybe aegerita* lectin AAL show that the molecular weight of AAL is 15.8 kDa and the molecular weight of AAL-2 is 43 kDa (Figure 5).
4. The carbohydrate spectrum analysis results of the separated *Agrocybe aegerita* lectin AAL-2 and the published *Agrocybe aegerita* lectin AAL are shown in Table 2 and table 3, respectively.

**Table 2. carbohydrate spectrum analysis results of AAL-2 (glycosyl structure affinity of top 18)**

| **order** | glycosyl structure |
|---|---|
| 1 | GlcNAcα1-4Galβ1-4GlcNAcβ1-3Galβ1-4GlcNAcβ1-3Galβ1-4GlcNAcβ-Sp0 |
| 2 | GlcNAcα1-3Galβ1-4GlcNAcβ-Sp8 |
| 3 | GlcNAcβ1-3(GlcNAcβ1-6)Galβ1-4GlcNAcβ-Sp8 |
| 4 | GlcNAcα1-4Galβ1-4GlcNAcβ1-3Galβ1-4Glcβ-Sp0 |
| 5 | GlcNAcβ1-3Galβ1-4GlcNAcβ-Sp0 |
| 6 | GlcNAcα1-6Galβ1-4GlcNAcβ-Sp8 |
| 7 | GlcNAcα1-4Galβ1-4GlcNAcβ-Sp0 |
| 8 | GlcNAcβ1-3Galβ1-4GlcNAcβ1-3Galβ1-4GlcNAcβ-Sp0 |
| 9 | GlcNAcβ1-3Galβ1-4GlcNAcβ-Sp8 |
| 10 | GlcNAcα1-4Galβ1-3GlcNAcβ-Sp0 |
| 11 | GlcNAcα1-4Galβ1-4GlcNAcβ1-3Galβ1-4GlcNAcβ-Sp0 |
| 12 | GlcNAcβ1-4GlcNAcβ-Sp10 |
| 13 | GalNAcβ1-3GalNAcα-Sp8 |
| 14 | GlcNAcα1-4Galβ1-4GlcNAcβ1-3Galβ1-4(Fucα1-3)GlcNAcβ1-3Galβ1-4(Fucα1-3)GlcNAcβ-Sp0 |
| 15 | GlcNAcβ1-3Galβ1-3GalNAcα-Sp8 |
| 16 | GlcNAcβ1-3Galβ1-4Glcβ-Sp0 |
| 17 | GlcNAcβ1-3Galβ-Sp8 |
| 18 | GlcNAcβ1-6(GlcNAcβ1-3)GalNAcα-Sp14 |

**Table 3. carbohydrate spectrum analysis results of AAL**

| **order** | glycosyl structure |
|---|---|
| **1** | **[3OSO3]Galb1-3GalNAca-Sp8** |
| **2** | **Gala1-3Galb1-4GlcNAcb1-2Mana1-3(Gala1-3Galb1-4GlcNAcb1-2Mana1-6)Manb1-4GlcNAcb1-4GlcNAcb-Sp20** |
| **3** | **Neu5Aca2-3Galb1-3GalNAca-Sp8** |
| **4** | **Neu5Aca2-3Galb1-4GlcNAcb1-2Mana1-3(Neu5Aca2-3Galb1-4GlcNAcb1-2Mana1-6)Manb1-4GlcNAcb1-4GlcNAcb-Sp12** |
| **5** | **[3OSO3]Galb1-4[6OSO3]GlcNAcb-Sp8** |
| **6** | **[3OSO3]Galb1-4[6OSO3]Glcb-Sp8** |
| **7** | **Neu5Aca2-3Galb1-3[6OSO3]GalNAca-Sp8** |
| **8** | **[3OSO3]Galb1-4GlcNAcb-Sp8** |
| **9** | **Neu5Aca2-3Galb1-3GalNAcb1-3Gala1-4Galb1-4Glcb-Sp0** |
| **10** | **Neu5Aca2-6Galb1-4GlcNAcb1-2Mana1-3(Neu5Aca2-3Galb1-4GlcNAcb1-2Mana1-6)Manb1-4GlcNAcb1-4GlcNAcb-Sp12** |
| **11** | **Neu5Aca2-3Galb1-4GlcNAcb1-3Galb1-4GlcNAcb1-3Galb1-4GlcNAcb-Sp0** |
| **12** | **[3OSO3]Galb1-3GlcNAcb-Sp8** |
| **13** | **[3OSO3]Galb1-4[6OSO3]GlcNAcb-Sp0** |
| **14** | **Neu5Ac(9Ac)a2-3Galb1-4GlcNAcb-Sp0** |
| **15** | **NeuAca2-3Galb1-4GlcNAcb1-3GalNAc-Sp14** |
| **16** | **Gala1-3Galb1-4GlcNacb1-3GalNaca-Sp14** |
| **17** | **[3OSO3]Galb1-4GlcNAcb-Sp0** |
| **18** | **Galb1-4GlcNAcb1-2Mana1-3(Galb1-4GlcNAcb1-2Mana1-6)Manb1-4GlcNAcb1-4GlcNAcb-Sp13** |

According to the above experimental results, we can see that the *Agrocybe aegeruta* lectin AAL in the existing techniques has a molecular mass of 15.8 kDa, which is a dimer; *Agrocybe aegeruta* lectin AAL-2 isolated in this invention has a molecular mass of 43 kDa, which is a monomer. The nucleotide sequence and amino acid sequence comparisons of the AAL and AAL-2 show that the nucleotide homology is 40% and the homology of amino acid is about 20%.The carbohydrate spectrum shows that the AAL-2 specifically binds to the glycosyl with N-Acetylglucosamine as the terminal, which is completely different from the carbohydrate spectrum of AAL. Therefore, the separated *Agrocybe aegerita* lectin AAL-2 in the invention is another kind of *Agrocybe aegerita* lectin completely different from AAL.

### Experiment example 2 The antitumor activity experiment of Agrocybe aegerita lectin AAL-2 protein

### 1. The inhibition effect of Agrocybe aegerita lectin AAL-2 on hepatocellular carcinoma cells in vitro

The experiment detects the killing activity of *Agrocybe aegerita* lectin AAL-2 (isolated protein in example 1) to the two tumor cell lines (H22, Huh7). H22 and Huh7 cell lines (purchased from ATCC) are cultured in RPMI1640 medium and DMEM medium (contains 10% fetal bovine serum, 100mg/mL streptomycin and 100U/mL penicillin). Different concentrations of *Agrocybe aegerita* lectin AAL-2 prepared in the example1 are added into tumor cells (final concentration is 0.6 µmol/L, 1.2 µmol/L, 2.4 µmol/L) and cultured for 24 hours and 36 hours, respectively. The treated cells are digested into single cell, stained with the Annexin V/PI dye and detected by the flow cytometry.

The detection results are shown in Figure 6, *Agrocybe aegerita* lectin AAL-2 can significantly cause two hepatoma cell lines apoptosis and thus kill the hepatoma cells.

### 2. The inhibition effect of Agrocybe aegerita lectin AAL-2 on the tumor growth of tumor bearing mice

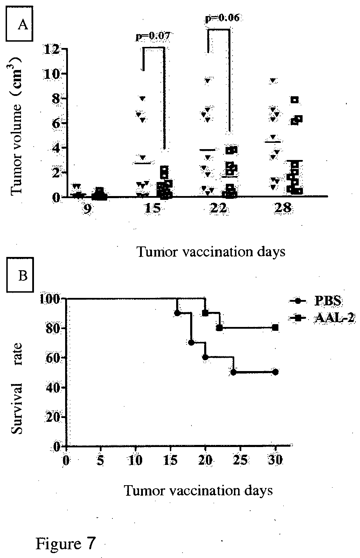

In this experiment, the BALB/c mice (6-8weeks old, 20 g) are used for the antitumour activity assay. 0.1 ml mouse hepatoma cell line H22 (1×10⁷ cells/mL) is subcutaneously inoculated into the oxter area of the BALB/c mice. 7 days after inoculation, inject the *Agrocybe aegerita* lectin AAL-2 by in situ tumour (protein isolated in example 1) (5 mg/kg, 0.1 ml/mouse). The control group is treated with 0.9%NaCI. Then take the injection every other day, a total of 7 injections, and record the size of the tumor tissue in mice. Tumor size = tumor tissue length L × width W²/2. The results are shown in figure 7. *Agrocybe aegerita* lectin AAL-2 could inhibit the tumor growth and prolong the survival time of tumor bearing mice.

The cell experiment results show that the *Agrocybe aegerita* lectin AAL-2 isolated in the present invention has the killing activity to liver cancer cells; animal experiments indicate that AAL-2 inhibits the tumor growth of the H22 tumor bearing mice and prolongs the survival time of the tumor bearing mice, which has a definite therapeutic effect on the tumor bearing mice.

### Experimental example 3 The carbohydrate binding specificity of Agrocybe aegerita lectin AAL-2

In order to identify the carbohydrate binding activity of *Agrocybe aegerita* lectin AAL-2, the *Agrocybe aegerita* lectin AAL-2 is used to detect 465 kinds of carbohydrate by the glycochip technology.

Firstly, the purified *Agrocybe aegerita* lectin AAL-2 (isolated in example 1) is labeled with the biotin and 200µg/mL AAL-2 incubates the glycochip, then the fluorescein labeled streptavidin combines with the biotin labeled AAL-2. Results are shown in Table 4 (different carbohydrate affinity of AAL-2 arranged from high to low (top of 23 carbohydrates)). *Agrocybe aegerita* lectin AAL-2 can specifically preferentially bind to carbohydrates with N-Acetylglucosamine as the terminal, which suggest that AAL-2 has the potential to developing into a probe that can recognize the N-Acetylglucosamine.

**Table 4**

| number | glycosyl structure | average | **StDEV^{b}** | **SEM^{c}** | % **CV^{d}** |
|---|---|---|---|---|---|
| 336 | GlcNAc α 1-4Gal β 1-4GlcNAc β 1-3Gal β 1-4GlcNAc β 1-3Gal β 1-4GlcNAc β -Sp0 | 30519 | 7420 | 3710 | 24 |
| 171 | GlcNAc α 1-3Gal β 1-4GlcNAc β -Sp8 | 24487 | 975 | 488 | 4 |
| 175 | GlcNAc β 1-3(GlcNAc β 1-6)Gal β 1-4GlcNAc β -Sp8 | 21115 | 5104 | 2552 | 24 |
| 339 | GlcNAc α 1-4Gal β 1-4GlcNAc β 1-3Gal β 1-4Glc β -Sp0 | 20868 | 2302 | 1151 | 11 |
| 179 | GlcNAc β 1-3Gal β 1-4GlcNAc β -Sp0 | 19530 | 398 | 199 | 2 |
| 172 | GlcNAc α 1-6Gal β 1-4GlcNAc β -Sp8 | 19311 | 1314 | 657 | 7 |
| 337 | GlcNAc α 1-4Gal β 1-4GlcNAc β -Sp0 | 19030 | 3387 | 1693 | 18 |
| 181 | GlcNAc β 1-3Gal β 1-4GlcNAc β 1-3Gal β 1-4GlcNAc β -Sp0 | 18737 | 6399 | 3199 | 34 |
| 180 | GlcNAc β 1-3Gal β 1-4GlcNAc β -Sp8 | 17289 | 2316 | 1158 | 13 |
| 338 | GlcNAc α 1-4Gal β 1-3GlcNAc β -Sp0 | 16141 | 3703 | 1851 | 23 |
| 341 | GlcNAc α 1-4Gal β 1-4GlcNAc β 1-3Gal β 1-4GlcNAc β -Sp0 | 11672 | 3034 | 1517 | 26 |
| 308 | GlcNAc β 1-4GlcNAc β -Sp10 | 11572 | 3025 | 1513 | 26 |
| 94 | GalNAc β 1-3GalNAc α -Sp8 | 11198 | 1039 | 520 | 9 |
| 340 | GlcNAc α 1-4Gal β 1-4GlcNAc β 1-3Gal β 1-4(Fuc α 1-3)GlcNAc β 1-3Gal β 1-4(Fuc α 1-3)GlcNAc β -Sp0 | 8758 | 558 | 279 | 6 |
| 88 | GlcNAc β 1-3Gal β 1-3GalNAc α -Sp8 | 8504 | 1554 | 777 | 18 |
| 182 | GlcNAc β 1-3GaI β 1-4Glc β -Sp0 | 8464 | 2012 | 1006 | 24 |
| 178 | GlcNAc β 1-3Gal β -Sp8 | 7339 | 1036 | 518 | 14 |
| 407 | GlcNAc β 1-6(GlcNAc β 1-3)GalNAc α -Sp14 | 6992 | 766 | 383 | 11 |
| 309 | GlcNAc β 1-4GlcNAc β -Sp12 | 6078 | 479 | 239 | 8 |
| 176 | GlcNAc β 1-3GalNAc α -Sp8 | 5896 | 261 | 131 | 4 |
| 177 | GlcNAc β 1-3GalNAc α -Sp14 | 5782 | 975 | 488 | 17 |
| 190 | GlcNAc β 1-6GalNAc α -Sp8 | 5571 | 2595 | 1297 | 47 |
| 17 | GlcNAc β -Sp8 | 5331 | 2017 | 1008 | 38 |

## Claims

1. An isolated cDNA having the nucleic acid sequence of SEQ ID NO: 1, said nucleic acid encoding a protein having the amino acid sequence of SEQ ID NO: 2.

2. The isolated cDNA according to claim 1, wherein the isolated cDNA is inserted in an expression vector.

3. The isolated cDNA according to claim 2, wherein the expression vector is transformed into a host cell.

4. An isolated protein a) which comprises amino acid SEQ ID NO: 2; b) which shares at least 90% identify with SEQ ID NO: 2; or c) which shares at least 80% identify with SEQ ID NO: 2.

5. The isolated protein according to claim 4; wherein the isolated protein is purified from *Agrocybe aegerita.*

6. The isolated protein according to claim 4, wherein the isolated protein is purified from the host cells of claim 3 that contains the expression vector of claim 2.

7. A pharmaceutical composition that comprises an effective dose of the isolated protein of claim 4 and pharmaceutically acceptable carriers.

8. The isolated protein of claim 4 for use as a medicament.

9. The isolated protein of claim 4 for use in the treatment of a tumors.

10. The isolated protein according to claim 9, for use in the treatment of a tumors by inhibiting the growth of tumor cells.

11. An in-vitro method for detecting N-Acetylglucosamine-associated carbohydrate, wherein the binding of isolated protein of claim 4 with N-Acetylglucosamine-associated carbohydrate is determined.

12. The method according to claim 11, wherein the isolated protein is labeled with a fluorescein.

## Patentansprüche

1. Isolierte cDNA mit der Nukleotidsequenz aus SEQ ID NO: 1, wobei diese Nukleinsäure ein Protein mit der Aminosäuresequenz aus SEQ ID NO: 2 kodiert.

2. Isolierte cDNA nach Anspruch 1, wobei die isolierte cDNA in einen Expressionsvektor eingesetzt wird.

3. Isolierte cDNA nach Anspruch 2, wobei der Expressionsvektor in eine Wirtszelle transformiert wird.

4. Isoliertes Protein, a) welches Aminosäure SEQ ID NO: 2 umfasst; b) welches mindestens 90 % Identifikation mit SEQ ID NO: 2 teilt; oder c) welches mindestens 80% Identifikation mit SEQ ID NO: 2 teilt.

5. Isoliertes Protein nach Anspruch 4, wobei das isolierte Protein aus *Agrocybe aegerita* aufgereinigt wird.

6. Isoliertes Protein nach Anspruch 4, wobei das isolierte Protein aus der Wirtszelle nach Anspruch 3, welche den Expressionsvektor nach Anspruch 2 enthält, aufgereinigt wird.

7. Pharmazeutische Zusammensetzung, welche eine effektive Dosis des isolierten Proteins nach Anspruch 4 und pharmazeutisch akzeptable Träger umfasst.

8. Isoliertes Protein nach Anspruch 4 zur Verwendung als Medikament.

9. Isoliertes Protein nach Anspruch 4 zur Verwendung in der Behandlung von Tumoren.

10. Isoliertes Protein nach Anspruch 9 zur Verwendung in der Behandlung von Tumoren durch Hemmung des Wachstums von Tumorzellen.

11. In-vitro-Verfahren zum Nachweis von N-Acetylglucosamin-assoziiertem Kohlenhydrat, wobei die Bindung des isolierten Proteins nach Anspruch 4 mit N-Acetylglucosamin-assoziiertem Kohlenhydrat bestimmt wird.

12. Verfahren nach Anspruch 11, wobei das isolierte Protein mit Fluorescein markiert ist.

## Revendications

1. ADNc isolé ayant la séquence d'acide nucléique de SEQ ID No : 1, ledit acide nucléique codant pour une protéine ayant la séquence d'acides aminés de SEQ ID No : 2.

2. ADNc isolé selon la revendication 1, dans lequel l'ADNc isolé est inséré dans un vecteur d'expression.

3. ADNc isolé selon la revendication 2, dans lequel le vecteur d'expression est transformé dans une cellule hôte.

4. Protéine isolée a) qui comprend la séquence d'acides aminés de SEQ ID No : 2 ; b) qui partage une identité d'au moins 90 % avec SEQ ID No : 2 ; ou c) qui partage une identité d'au moins 80 % avec SEQ ID No : 2.

5. Protéine isolée selon la revendication 4, dans laquelle la protéine isolée est purifiée à partir d'*Agrocybe aegerita.*

6. Protéine isolée selon la revendication 4, dans laquelle la protéine isolée est purifiée à partir des cellules hôtes selon la revendication 3 qui contiennent le vecteur d'expression selon la revendication 2.

7. Composition pharmaceutique qui comprend une dose efficace de la protéine isolée selon la revendication 4 et des véhicules pharmaceutiquement acceptables.

8. Protéine isolée selon la revendication 4 pour son utilisation à titre de médicament.

9. Protéine isolée selon la revendication 4 pour son utilisation dans le traitement d'une tumeur.

10. Protéine isolée selon la revendication 9, pour son utilisation dans le traitement d'une tumeur par inhibition de la croissance des cellules tumorales.

11. Méthode in vitro pour détecter un glucide associé à une N-acétylglucosamine, dans laquelle la liaison de la protéine isolée selon la revendication 4 avec un glucide associé à une N-acétylglucosamine est déterminée.

12. Méthode selon la revendication 11, dans laquelle la protéine isolée est marquée avec une fluorescéine.
